# EUROPEAN PATENT APPLICATION

(11) **EP 3 805 208 A1**
(43) Date of publication of application: **14.04.2021**
(21) Application number: 20758997.9
(22) Date of filing: 18.02.2020
(51) Int. Cl.: C07D 317/36, C07D 317/38

(54) **METHOD FOR PREPARING ALKYLENE CARBONATE FROM EPOXYALKANE AND CARBON DIOXIDE**

(30) Priority: 19.02.2019 CN 201910121914
(71) Applicant: Shandong Shida Shenghua Chemical Group Co., Ltd, Shandong 257503 (CN)
(72) Inventor: GUO, Jianjun, Shandong 257503 (CN); LI, Xin, Shandong 257503 (CN); MA, Guo, Shandong 257503 (CN); LI, Guangke, Shandong 257503 (CN); XUE, Maowei, Shandong 257503 (CN)
(74) Representative: Ter Meer Steinmeister & Partner
(86) International application number: PCT/CN2020/075759
(87) International publication number: WO 2020/169032

(57) **Abstract**

The present disclosure relates to a method for preparing alkylene carbonate from alkylene oxide and carbon dioxide. The method comprises: contacting a raw material comprising alkylene oxide and carbon dioxide with a catalyst to produce alkylene carbonate; wherein the catalyst is at least one selected from the complexes formed from multidentate ligand and zinc; wherein the multidentate ligand comprises triphenylphosphine-based compound and salicylic acid-based compound; a molar ratio of the zinc, the triphenylphosphine-based compound and the salicylic acid-based compound in the catalyst is any ratio in a range of 5:1-50: 1-50. The beneficial effects achieved are as follows: the method adopts a complex catalyst system formed from multidentate ligand and zinc. The catalyst achieved 99.8% conversion efficiency of alkylene oxide, 99.5% selectivity of alkylene carbonate, and thus achieved good technical effect.

## Description

### TECHNICAL FIELD

The present disclosure relates to a method for preparing alkylene carbonate, in particular to a method for preparing the alkylene carbonate from alkylene oxide and carbon dioxide.

### BACKGROUND

The alkylene carbonate is an excellent solvent, a fine chemical intermediate, and a potential basic raw material for organic chemicals. Meanwhile, CO₂ is a greenhouse gas. How to effectively fix CO₂ becomes one of the most challenging problems in the century, while it is a good fixation method for synthesizing alkylene carbonate by a reaction between alkylene oxide and CO₂. With increasing interest in the co-production of dimethyl carbonate and diols starting from alkylene carbonates recently, the fixation of CO₂ by cyclic carbonates has also been paid increasing attention.

It has been reported that there are various catalyst systems for an esterification reaction of CO₂ and EO, comprising ammonium onium halides, phosphonium onium halides, tributyl methyl phosphonium iodide, metal halides (such as KBr, ZnCl₂, etc.), triphenylphosphine-based and pyridine-based ionic liquids, metal complexes, amines, and polymer supported catalysts, etc. There are some disadvantages in these catalyst systems, such as low reaction activity or yellowing of obtained product, thereby resulting in degraded quality of the product. US2907771 discloses the followings: Olin Mathieson, a US company, found that an EC solution of alkaline earth metal salt CaCl₂ has a better catalytic activity in a cycloaddition reaction of EO, and an EC yield of 68.4% is achieved in a continued reaction at 800 Psi and 190 °C. US7488835 disclosed the followings: Shell Group of Companies studied the effect of the types of alkali metal and complex on the catalysts for catalyzing EC synthesis reaction from EO, and found that the best effect was achieved when the catalyst system was KI/18-crown-6, and an EO conversion efficiency of 84% and a EC selectivity of 98% were achieved under the reaction condition of 2MPa, 80 °C for 1 h. CN1995032 discloses the followings: ionic liquid of 1-methyl-3-butylimidazole bromide was used as catalyst, and an EC yield of 92.2 % was achieved under the reaction condition of EO/Cat = 208, 100 °C and 2.0MPa for 0.7 h .

We herein prepared a catalyst system with multidentate complex compound, which comprises triphenylphosphine group, salicylic acid group and zinc, and achieved a catalyst with good activity.

### SUMMARY

In view of the above disadvantages of the prior art, the object of present disclosure is to provide a method for preparing alkylene carbonate from alkylene oxide and carbon dioxide, which is characterized by high catalyst activity.

The disclosure provides a method for preparing alkylene carbonate from alkylene oxide and carbon dioxide, comprising:
contacting a raw material comprising alkylene oxide and carbon dioxide with a catalyst to produce alkylene carbonate;
wherein the catalyst is at least one selected from complexes formed from multidentate ligand and zinc;
wherein the multidentate ligand comprises triphenylphosphine-based compound and salicylic acid-based compound; and
wherein, a molar ratio of the triphenylphosphine-based compound, the salicylic acid-based compound and zinc in the catalyst, is any ratio in a range of 5: 1-50: 1-50.

Preferably, reaction conditions are as follows: a reaction temperature is any value in a range from 60°C to 200°C, a reaction pressure is any value in a range from 0.1 MPa to 10.0MPa, and a mass ratio of the catalyst to the alkylene oxide in the raw material is any ratio within the range from 0.001:1 to 1: 1.

Preferably, the molar ratio of the zinc, the triphenylphosphine-based compound and the salicylic acid-based compound is in a range of 5: 2-10: 2-5.

Preferably, the molar ratio of the zinc, the triphenylphosphine-based compound and the salicylic acid-based compound, is any ratio selected from 5:1:1, 5:3:5, 5:6:5, 5:10:2, 5:2:3, 5:4:2, 5:7:2, 5:2:2, and 5:50:50, or is any ratio in a range between any two ratios above.

Preferably, the triphenylphosphine-based compound is at least one selected from compounds having a chemical formula shown in formula I: 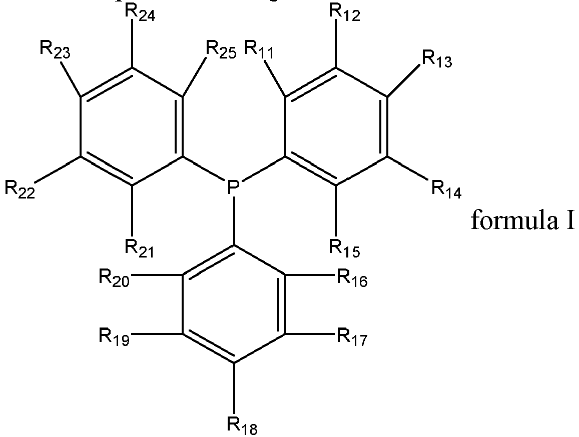 wherein, in the formula I, R₁₁, R₁₂, R₁₃, R₁₄, R₁₅, R₁₆, R₁₇, R₁₈, R₁₉, R₂₀, R₂₁, R₂₂, R₂₃, R₂₄ and R₂₅ independently from each other are H or C₁-C₁₂ alkyl.

Preferably, R₁₁, R₁₆ and R₂₁ are the same group selected from F, Cl, Br, I, methyl, ethyl group and propyl;
R₁₂, R₁₇ and R₂₂ are the same group selected from H, F, Cl, Br, I, methyl, ethyl and propyl;
R₁₃, R₁₈ and R₂₃ are the same group selected from H, F, Cl, Br, I, methyl, ethyl and propyl;
R₁₄, R₁₉ and R₂₄ are the same group selected from H, F, Cl, Br, I, methyl, ethyl and propyl;
R₁₅, R₂₀ and R₂₅ are the same group selected from H, F, Cl, Br, I, methyl, ethyl and propyl.

Preferably, the salicylic acid-based compound is at least one selected from compounds having a chemical formula shown in formula II: 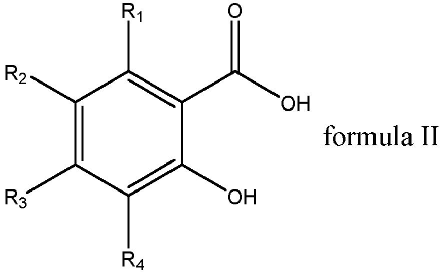 wherein, R₁, R₂, R₃ and R₄ independently from each other are H or C₁∼C₁₂ alkyl.

Preferably, the triphenylphosphine-based compound is at least one selected from triphenylphosphine, alkyl triphenylphosphine and halogen triphenylphosphine.

Preferably, the triphenylphosphine-based compound is at least one selected from triphenylphosphine, 1-methyltriphenylphosphine and 2-methyl triphenylphosphine.

Preferably, the salicylic acid-based compound is at least one selected from salicylic acid, 3-methyl salicylic acid and 4-methyl salicylic acid.

Preferably, the alkylene oxide is at least one selected from ethylene oxide, propylene oxide, epichlorohydrin oxide and butylene oxide.

Preferably, the source of zinc is at least one selected from an oxide of zinc and an inorganic salt of zinc.

Preferably, the source of zinc is at least one selected from zinc chloride, zinc bromide, zinc sulfate, zinc nitrate and zinc oxide.

Preferably, the reaction temperature is in a range from 80∼160°C, the reaction pressure is in a range from 0.5 MPa to 8.0MPa, and a mass ratio of the catalyst to the alkylene oxide is in a range from 0.005:1 to 0.5:1.

Preferably, the upper limit of the reaction temperature is 80°C, 100°C, 120°C, 140°C, 160°C, 180°C or 200°C, while the lower limit of the reaction temperature is 60°C, 80°C, 100°C, 120°C, 140°C, 160°C or 180°C. Preferably, the upper limit of the reaction pressure is 0.2 MPa, 0.5MPa, 0.8MPa, 1.0MPa, 2.0MPa, 3.0MPa, 4.0MPa, 5.0MPa, 6.0MPa, 7.0MPa, 8.0MPa, 9.0 MPa, or 10.0 MPa, while the lower limit is 0.1MPa, 0.2MPa, 0.5MPa, 0.8MPa, 1.0MPa, 2.0MPa, 3.0MPa, 4.0MPa, 5.0MPa, 6.0MPa, 7.0MPa, 8.0MPa, or 9.0MPa.

Preferably, the reacting step comprises: sequentially adding the catalyst and the alkylene oxide in a reactor, and feeding carbon dioxide gas to the reactor to perform the reaction under the reaction conditions.

Preferably, a method for preparing the above catalyst comprises the following steps:
1) mixing a compound comprising zinc element with a salicylic acid-based compound, adding a solvent therein, and heating to reflux, thereby obtaining a mixture;
2) mixing the mixture with the triphenylphosphine-based compound, heating to reflux, and removing the solvent to obtain a solid product which is the catalyst;
wherein, a molar ratio of the compound comprising the zinc element, salicylic acid-based compound and the triphenylphosphine-based compound is any ratio in a range of 5: 1-50 : 1-50;
wherein, the mole number of the compound comprising zinc element is counted based on the mole number of the zinc element.

Preferably, during the preparation of the catalyst, a molar ratio of the compound comprising the zinc element, the triphenylphosphine-based compound and the salicylic acid-based compound used in steps 1) and 2) is any ratio selected from 5:1:1, 5:3:5, 5:6:5, 5:10:2, 5:2:3, 5:4:2, 5:7:2, 5:2:2 and 5:50:50, or any ratio in the range between any two above ratios.

Preferably, during the preparation of the catalyst, the compound comprising zinc element used in step 1) is at least one of oxide of zinc and inorganic salt of zinc.

Preferably, the solvent is at least one selected from methanol, ethanol and diethyl ether.

Preferably, in steps 1) and 2), reflux time is in a range from 4hours to 8 hours.

In the present disclosure, the alkyl in "C₁∼C₁₂ alkyl" refers to an alkyl having 1∼12 carbon atoms.

In present disclosure, "alkyl" refers to a group obtained from an alkane by losing one H atom.

The beneficial effects achieved by the present disclosure are as follows: the method adopts a complex catalyst system formed from multidentate ligand and zinc, wherein the multidentate ligand comprises triphenylphosphine-based compound and salicylic acid-based compound; a molar ratio of the triphenylphosphine-based compound, the salicylic acid-based compound and the zinc in the catalyst is any ratio in the range of 5: 1-50 : 1-50. The catalyst in the present disclosure can catalyze reaction at any temperature in a range from 60°C to 200°C and any pressure in a range from 0.1 MPa to 10.0 MPa, with a mass ratio of the catalyst to the alkylene oxide being in a range from 0.001:1 to 1:1. The reaction was maintained for 3 hours to obtain 99.8% conversion efficiency of alkylene oxide, 99.5% selectivity of alkylene carbonate, and thus a good technical effect was achieved. The method for preparing the catalyst system is simple, with low cost, and the catalyst exhibits good activity and could be popularized to the industrial production of alkylene carbonate.

### DETAILED DESCRIPTION

The preferred examples of the present disclosure are described below. It should be understood that the preferred examples described herein are only used to illustrate and explain the present disclosure and are not to be construed as limiting the present disclosure.

In these examples, the conversion efficiency of alkylene oxide = (the mole number of alkylene oxide before reaction - the mole number of alkylene oxide after reaction) / the mole number of alkylene oxide before reaction * 100%.

The selectivity of alkylene carbonate = the mole number of alkylene carbonate produced / the total mole of product produced.

### Example 1

0.5mol zinc oxide and 0.5mol salicylic acid were mixed, and 200ml ethanol was then added therein. They were stirred and heated to reflux for 6h, and then 0.3mol triphenylphosphine was added therein and mixed. Then the resulting mixture was stirred and heated to reflux for 6h, then cooled and filtered to obtain a catalyst product A.

### Example 2

0.5mol zinc bromide and 0.5mol salicylic acid were mixed, and 200ml ethanol was then added therein. They were stirred and heated to reflux for 6h, and then 0.6mol triphenylphosphine was added therein and mixed. Then the resulting mixture was stirred and heated to reflux for 6h, then cooled and filtered to obtain a catalyst product B.

### Example 3

0.5mol zinc chloride and 0.2mol salicylic acid were mixed, and 200ml ethanol was then added therein. They were stirred and heated to reflux for 6h, and then 1mol triphenylphosphine was added therein and mixed. Then the resulting mixture was stirred and heated to reflux for 6 hours, then cooled and filtered to obtain a catalyst product C.

### Example 4

0.5mol zinc chloride and 0.3mol salicylic acid were mixed, and 200ml ethanol was then added therein. They were stirred and heated to reflux for 6h, and then 0.2mol tris(1-methylphenyl)phosphine was added therein and mixed. Then the resulting mixture was stirred and heated to reflux for 6h, then cooled and filtered to obtain a catalyst product D.

### Example 5

0.5mol zinc sulfate and 0.2mol salicylic acid were mixed, and 200ml ethanol was then added therein. They were stirred and heated to reflux for 6h, and then 0.4mol tris(2-methylphenyl)phosphine was added therein and mixed. Then the resulting mixture was stirred and heated to reflux for 6h, then cooled and filtered to obtain a catalyst product E.

### Example 6

0.5mol zinc chloride and 0.2mol 3-methyl salicylic acid were mixed, and then 200ml ethanol was then added therein. They were stirred and heated to reflux for 6h, and then 1mol triphenylphosphine was added therein and mixed. Then the resulting mixture was stirred and heated to reflux for 6h, then cooled and filtered to obtain a catalyst product F.

### Example 7

0.5mol zinc chloride and 0.2mol 4-methyl salicylic acid were mixed, and 200ml ethanol was then added therein. They were stirred and heated to reflux for 6h, and then 0.7mol triphenylphosphine was added therein and mixed. Then the resulting mixture was stirred and heated to reflux for 6h, then cooled and filtered to obtain a catalyst product G.

### Example 8

0.5mol zinc chloride and 0.2mol salicylic acid were mixed, and 200ml ethanol was then added therein. They were stirred and heated to reflux for 6h, and then 0.2mol triphenylphosphine was added therein and mixed. Then the resulting mixture was stirred and heated to reflux for 6h, then cooled and filtered to obtain a catalyst product H.

### Comparative Example 1

0.5mol zinc bromide and 0.2mol salicylic acid were mixed, and then 50ml ethanol was added therein. Then the resulting mixture was stirred and heated to reflux for 6h, then cooled and filtered to obtain a solid intermediate product, thereby obtaining a catalyst product I.

### Comparative Example 2

0.5mol zinc bromide and 0.2mol triphenylphosphine were mixed, and then 50ml ethanol was added therein. Then the resulting mixture was stirred and heated to reflux for 6h, then cooled and filtered to obtain a catalyst product J.

### Comparative Example 3

0.2mol salicylic acid and 0.2mol triphenylphosphine were mixed, and 50ml ethanol was then added therein. Then the resulting mixture was stirred and heated to reflux for 6h, and the ethanol was removed to obtain a catalyst product K.

Comparing three Comparative Examples with Example 8, in the Comparative Example 1, two components were used to obtain catalyst product I, and the catalyst achieved 28.6% conversion efficiency of ethylene oxide (C_{EO}) and 24.8% selectivity of an ethylene carbonate (S_{EC}), which are shown in table below; in the Comparative Example 2, two components were used to obtain catalyst product J, and the catalyst achieved 68% conversion efficiency of ethylene oxide (C_{EO}) and 99.1% selectivity of an ethylene carbonate (S_{EC}), which are shown in table below; in the Comparative Example 3, two components were used to obtain catalyst product K, and the catalyst achieved 15.2% conversion efficiency of ethylene oxide (C_{EO}), and 43.3% selectivity of ethylene carbonate (S_{EC}). The results show a significant difference from that of Example 8 (the catalyst in Example 8 achieved 99.7% conversion efficiency of ethylene oxide (C_{EO}) and 99.6% selectivity of ethylene carbonate (S_{EC}).

### Example 9

0.05g catalyst A and 20mL (0.4mol) ethylene oxide were added into a 100 mL stainless steel autoclave in sequence, then the autoclave was sealed, carbon dioxide with proper pressure was filled therein, the temperature was slowly raised to 120°C by a temperature controller, then the reaction pressure was controlled to be 2.0 MPa, and the reaction was carried out for 2.0 h. After the reaction ended, the reactor was cooled, excessive CO₂ was slowly discharged, the reactor was opened, and a small amount of sample was taken for chromatographic analysis. It was tested that the conversion efficiency of ethylene oxide (C_{EO}) was 99.8% and the selectivity of ethylene carbonate (S_{EC}) was 99.5%.

### Example 10

0.05g catalyst A and 20 mL (0.4mol) propylene oxide were added into a 100mL stainless steel autoclave in sequence, then the autoclave was sealed, carbon dioxide with proper pressure was filled, the temperature was slowly raised to 120 °C by a temperature controller, then the reaction pressure was controlled to be 2.0 MPa, and the reaction was carried out for 2.0 h. After the reaction ended, the reactor was cooled, excessive CO₂ was slowly discharged, the reactor was opened, and a small amount of sample was taken for chromatographic analysis. It was tested that the conversion efficiency of propylene oxide was 99.8% and the selectivity of propylene carbonate (S_{EC}) was 99.9%.

### Example 11

0.05g catalyst A and 20 mL (0.4mol) epichlorohydrin were added into a 100mL stainless steel autoclave in sequence, then the autoclave was sealed, carbon dioxide with proper pressure was filled, the temperature was slowly raised to 120°C by a temperature controller, then the reaction pressure was controlled to be 2.0 MPa, and the reaction was carried out for 2.0 h. After the reaction ended, the reactor was cooled, excessive CO₂ was slowly discharged, then the reactor was opened, and a small amount of sample was taken for chromatographic analysis. It was tested that the conversion efficiency of epichlorohydrin was 99.9%, and the selectivity of chlorinated propylene carbonate was 99.7%.

### Examples 12-26

The catalytic reaction of ethylene oxide and carbon dioxide was carried out under the same reaction conditions as those in Example 9, except for the catalyst sample used, the amount thereof and the reaction pressure. The results obtained were shown in Table 1.

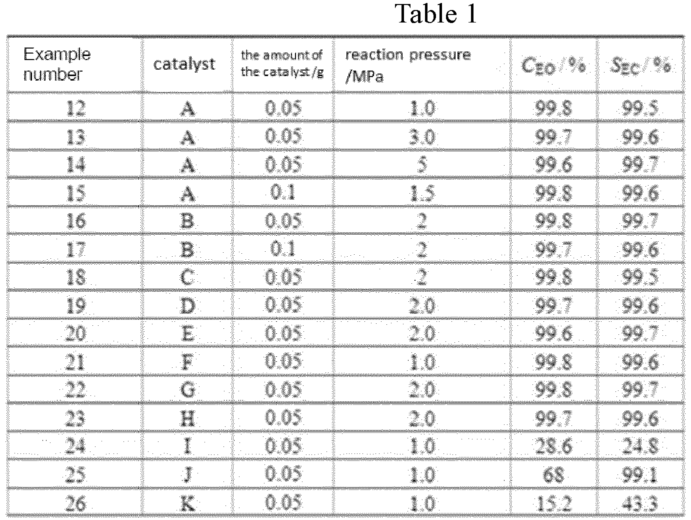

### Examples 27-34

The catalytic reaction of propylene oxide as raw material and carbon dioxide was carried out under the same reaction conditions as those in Example 10, except that the catalyst sample used was changed. The reaction results obtained were shown in Table 2.

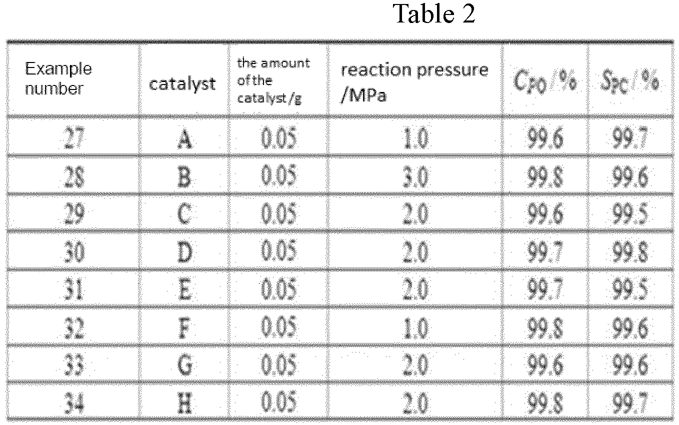

By comparisons, it can be seen that the catalysts in examples of the present disclosure exhibit improved activity or selectivity.

### Example 35

0.5mol zinc oxide and 0.1mol salicylic acid were mixed, and 200ml ethanol was then added therein. They were stirred and heated to reflux for 6h, and then 0.1mol triphenylphosphine was added therein and mixed. Then the resulting mixture were stirred and heated to reflux for 6h, then cooled and filtered to obtain a catalyst product L.

0.05g catalyst L and 20mL (0.4mol) ethylene oxide were added into a 100 mL stainless steel autoclave in sequence, the autoclave was sealed, carbon dioxide with proper pressure was filled, the temperature was slowly raised to 120°C by a temperature controller, then the reaction pressure was controlled to be 2.0 MPa, and the reaction was carried out for 2.0 h. After the reaction ended, the reactor was cooled, excessive CO₂ was slowly discharged, the reactor was opened, and a small amount of sample was taken for chromatographic analysis. The conversion efficiency of ethylene oxide (C_{EO}) was above 99.5% and the selectivity of ethylene carbonate (S_{EC}) was above 99%.

### Example 36

0.5mol zinc oxide and 5mol salicylic acid were mixed, and then 200ml ethanol was added therein. They were stirred and heated to reflux for 6h, and 5mol triphenylphosphine was added therein and mixed. Then, the resulting mixture were stirred and heated to reflux for 6h, and then cooled and filtered to obtain a catalyst product M.

0.05g catalyst M and 20 mL (0.4mol) ethylene oxide were added into a 100 mL stainless steel autoclave in sequence, the autoclave was sealed, carbon dioxide with proper pressure was filled, the temperature was slowly raised to 140 °C by a temperature controller, then the reaction pressure was controlled to be 2.0MPa, and the reaction was carried out for 2.0h. After the reaction ended, the reactor was cooled, excessive CO₂ was slowly discharged, the reactor was opened, and a small amount of sample was taken for chromatographic analysis. It was tested that the conversion efficiency of ethylene oxide (C_{EO}) was above 99%, and the selectivity of ethylene carbonate (S_{EC}) was above 99%.

In view of the above, although the present disclosure has been described with reference to some preferred examples, it should be understood that the preferred examples described herein are only used to illustrate and are not to be construed as limiting the present disclosure. Various changes and modifications made under the teachings of the present disclosure are equivalent to equivalent examples, without departing from the spirit and scope of the present disclosure.

## Claims

1. A method for preparing alkylene carbonate from alkylene oxide and carbon dioxide, **characterized in that**, the method comprises: contacting a raw material comprising alkylene oxide and carbon dioxide with a catalyst to produce alkylene carbonate;
wherein, the catalyst is at least one selected from complexes formed from multidentate ligand and zinc;
the multidentate ligand comprises triphenylphosphine-based compound and salicylic acid-based compound; and
a molar ratio of the zinc, the triphenylphosphine-based compound and the salicylic acid-based compound in the catalyst is any ratio within the range of 5: 1-50 : 1-50.

2. The method for preparing alkylene carbonate from alkylene oxide and carbon dioxide according to claim 1, **characterized in that**, reaction conditions are as follows:
a reaction temperature is any value in a range from 60°C to 200 °C, and a reaction pressure is any value in a range from 0.1 MPa tol0.0 MPa;and a mass ratio of the catalyst to the alkylene oxide in the raw material is any ratio in a range from 0.001:1 to 1: 1.

3. The method for preparing alkylene carbonate from alkylene oxide and carbon dioxide according to claim 1, **characterized in that**, the molar ratio of zinc, the triphenylphosphine-based compound and the salicylic acid-based compound in the catalyst is any ratio in a range of 5: 2-10 : 2-5.

4. The method for preparing alkylene carbonate from alkylene oxide and carbon dioxide according to claim 1, **characterized in that**, the triphenylphosphine-based compound is at least one selected from compounds having a chemical formula shown in formula I: 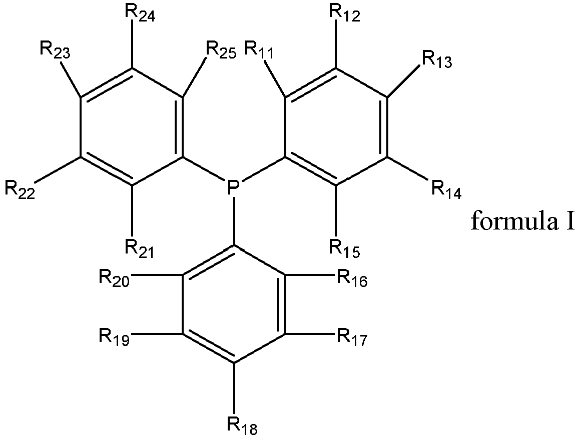 wherein, in Formula I, R₁, R₁₂, R₁₃, Ri4, R₁₅, R₁₆, R₁₇, R₁₈, R₁₉, R₂₀, R₂₁, R₂₂, R₂₃, R₂₄ and R₂₅ independently from each other are H or C₁-C₁₂ alkyl.

5. The method for preparing alkylene carbonate from alkylene oxide and carbon dioxide according to claim 4, **characterized in that**,
R₁₁, R₁₆ and R₂₁ are the same group selected from F, Cl, Br, I, methyl, ethyl and propyl;
R₁₂, R₁₇ and R₂₂ are the same group selected from H, F, Cl, Br, I, methyl, ethyl and propyl;
R₁₃, R₁₈ and R₂₃ are the same group selected from H, F, Cl, Br, I, methyl, ethyl and propyl;
R₁₄, R₁₉ and R₂₄ are the same group selected from H, F, Cl, Br, I, methyl, ethyl and propyl; and
R₁₅, R₂₀ and R₂₅ are the same group selected from H, F, Cl, Br, I, methyl, ethyl and propyl.

6. The method for preparing alkylene carbonate from alkylene oxide and carbon dioxide according to claim 1, **characterized in that**, the salicylic acid-based compound is at least one selected from compounds having a chemical formula shown in formula II: 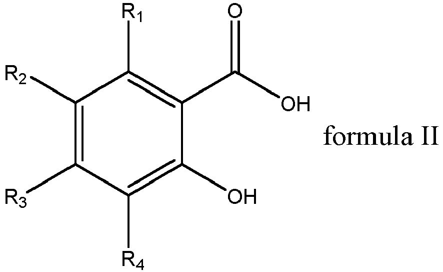 wherein, R₁, R₂, R₃ and R₄ independently from each other are H or C₁∼C₁₂ alkyl.

7. The method for preparing alkylene carbonate from alkylene oxide and carbon dioxide according to claim 1, **characterized in that**, the source of zinc is at least one selected from an oxide of zinc and an inorganic salt of zinc.

8. The method for preparing alkylene carbonate from alkylene oxide and carbon dioxide according to claim 1, **characterized in that**, the source of zinc is at least one selected from zinc chloride, zinc bromide, zinc sulfate and zinc oxide.

9. The method for preparing alkylene carbonate from alkylene oxide and carbon dioxide according to claim 1, **characterized in that**, the alkylene oxide is at least one selected from ethylene oxide, propylene oxide, epichlorohydrin and butylene oxide.

10. The method for preparing alkylene carbonate from alkylene oxide and carbon dioxide according to claim 1, **characterized in that**, reaction conditions are as follows:
a reaction temperature is any value in a range from 80°C to 160 °C, and a reaction pressure is any value in a range from 0.5 MPa to 8.0 MPa; and
a mass ratio of the catalyst to the alkylene oxide in the raw material is any ratio in a range from 0.005: 1 to 0.5: 1.

11. The method for preparing alkylene carbonate from alkylene oxide and carbon dioxide according to claim 1, **characterized in that**, the reacting step comprises: sequentially adding the catalyst and the alkylene oxide in a reactor, and feeding carbon dioxide gas to the reactor to perform the reaction under the reaction conditions.

12. The method for preparing alkylene carbonate from alkylene oxide and carbon dioxide according to claim 1, **characterized in that**, a method for preparing the catalyst comprises following steps:
1) mixing a compound comprising zinc element with a salicylic acid-based compound, adding a solvent therein, and heating to reflux to obtain a mixture;
2) mixing the mixture with a triphenylphosphine-based compound, heating to reflux, and removing the solvent to obtain a solid product which is the catalyst;
wherein, a molar ratio of the compound comprising the zinc element, the salicylic acid-based compound and triphenylphosphine-based compound is any ratio in a range of 5: 1-50: 1-50;
wherein, the mole number of the compound comprising zinc element is counted based on the mole number of the zinc element.

13. The preparation method according to claim 12, **characterized in that**, the solvent is at least one selected from alcohols and ethers.

14. The preparation method according to claim 12, **characterized in that**, the solvent is at least one selected from methanol, ethanol and diethyl.

15. The preparation method according to claim 12, **characterized in that**, in steps 1) and 2), reflux time is in a range from 4 hours to 8 hours.
